# EUROPEAN PATENT APPLICATION

(11) **EP 1 987 801 A2**
(43) Date of publication of application: **05.11.2008**
(21) Application number: 08155073.3
(22) Date of filing: 24.04.2008
(51) Int. Cl.: A61F 2/01

(54) **Retrievable Medical Filter with Diverse Baskets**

(30) Priority: 01.05.2007 US 742760
(71) Applicant: Cordis Corporation, Miami Lakes, FL 33014 (US)
(72) Inventor: Silver, James H., Palo Alto, California 94306 (US)
(74) Representative: Belcher, Simon James

(57) **Abstract**

An elongated medical filter is radially expandable and compressible and is intended for placement inside a tubular body passage, such as a blood vessel, of a patient. In expanded form, the medical filter has a spine with a retrieval element and a pair of longitudinally spaced filter baskets, one of the filter baskets having a plurality of independent legs and the other of the filter baskets having a plurality of legs with interconnecting struts.

## Description

The present invention relates to medical filters which are intended to be placed inside a blood vessel or other body passage for the purpose of intercepting thrombus or particles.

Medical filters, including vena cava filters, are emplaced inside blood vessels or other body passages. When used in a blood vessel, a medical filter should be effective to entrap thrombus, clots or other dangerous coagulations or particulate matter in the blood while allowing free flow of blood through the filter in the vessel. There are several other characteristics which are generally desirable in medical filters. For example, the filter should be of a design which can be placed in the blood vessel or body passage with minimal trauma to the patient and of a design which does not become entangled during its deployment. If it is desired to remove the filter after it has been implanted, it should be removable with minimal trauma to the vessel or body passage. It is also desirable that the filter be adapted to center in the vessel and to properly adjust to the size of the vessel, that the filter resist migration within the vessel, that the filter remain effective during its time in place, i.e., resist occlusion, and that the filter resist tilting or other unwanted movement within the vessel.

Although vena cava and other medical filters are known in the art and have been found to be effective, the requirements of such filters can be high and there remains room for improved designs. In particular, there remains room for improved filters which exhibit the above mentioned characteristics and yet can be economically manufactured. Accordingly, the present invention provides a medical filter which is suitable for placement in a blood vessel or body passage with minimal trauma to the blood vessel or body passage and is unlikely to become entangled during its deployment. The filter of this invention has a design which facilitates its removal from blood vessel or body passage even after an extended duration. Further more, the present invention provides a medical filter which is well adapted to center in the vessel and to properly adjust to the size of the vessel. The filter is designed to resist tilting and/or migration within the vessel. The filter is of a design which should be effective for a long duration without occlusion.

In accordance with the present invention, a preferred embodiment of the present invention is an elongated medical filter which has a longitudinal axis, is radially expandable and compressible and is intended for placement inside a tubular body passage, such as a blood vessel, of a patient. In expanded form, the medical filter comprises:
A. a spine extending along the longitudinal axis of the filter and having a retrieval element at one end thereof;
B. a first filter basket attached to said spine and comprising a plurality of discrete legs with free ends; and
C. a second filter basket attached to said spine longitudinally spaced from said first filter basket and comprising a plurality of legs with free ends and further with interconnecting struts extending between the legs.

In another preferred embodiment of the present invention, at least some of the legs of one or both of the first and second filter baskets have reentrantly bent end segments. In one embodiment of the present invention, the spine has a single retrieval element at one end thereof and the first filter basket is attached to the spine proximate to the retrieval element while the second filter basket is attached to the spine distal to the retrieval element.

Further understanding of the present invention will be had from the following description taken in conjunction with the accompanying drawings and appended claims.

Embodiments of the invention will now be described by way of example with reference to the accompanying drawings, in which:
FIG.1 is a perspective view of a preferred embodiment of a medical filter of the present invention shown in expanded form;
FIG. 2 is a perspective view of an alternative preferred embodiment of the present invention shown in expanded form; and
FIG. 3 is a perspective view, broken away, showing an alternative preferred embodiment of an arm with a reentrently bent end segment

The following description of the preferred embodiments of the present invention is intended to be merely illustrative in nature, and as such, is not intended to limit in any way the present invention, its application, or uses. Numerous modifications may be made by those skilled in the art without departing from the scope of the invention. For example, the preferred embodiments of the present invention are described in conjunction with a blood vessel but the preferred embodiments may also be used in other body passages.

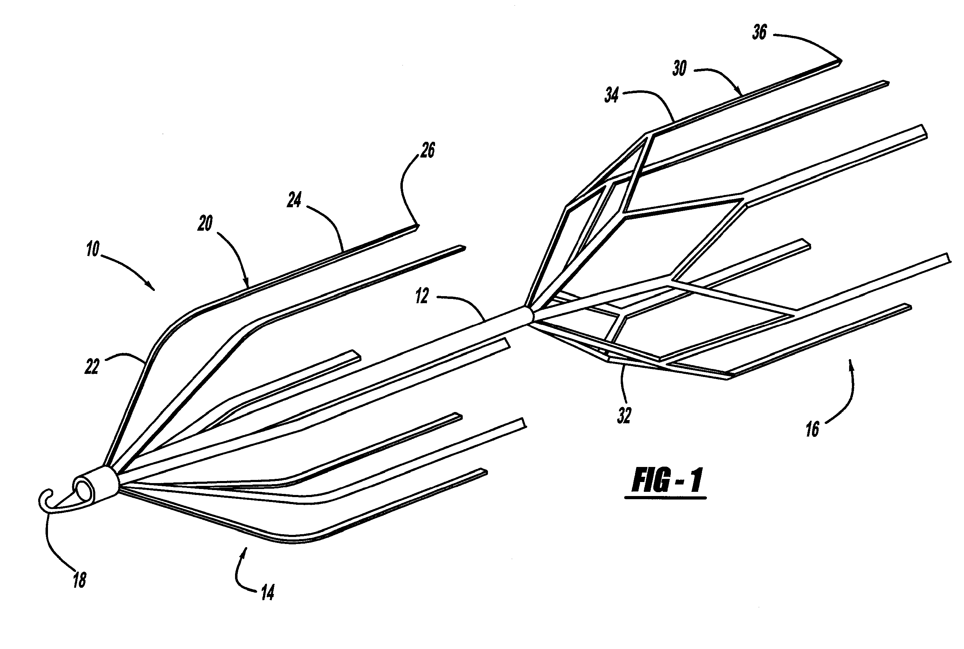

Now referring to Figure 1, a preferred embodiment of a medical filter of the present invention is shown and indicated generally by the numeral 10. Filter 10 is made of a resilient material, preferably a shape memory material, which tends to expand to the form illustrated in Figure 1 but can be compressed radially to a smaller diameter to be carried in the lumen of a suitable delivery catheter (not shown) as is conventional in the art.

Generally speaking medical filter 10 is an elongated structure with an axial spine 12 extending radially centrally along it longitudinal axis. Attached to spine 12 and longitudinally spaced thereon are a pair of filter baskets, first filter basket 14 and second filter basket 16, and a retrieval element 18 which is located at one longitudinal end thereof.

First filter basket 14 has a plurality of independent or discrete legs 20. Each leg 20 has a radially central end attached to, or formed with, spine 12 and a segment 22 extending radially outwardly and axially from spine 12 to a generally axially extending portion 24 with free end 26. Portions 24 of legs 20 extend axially generally parallel to each other and to the longitudinal axis of medical filter 10. Thus, each leg 20 is independent of each other leg 20 without interconnecting struts or the like. This structure is advantageous in that it minimizes chances of legs 20 becoming entangled with spine 12 when they expand radially outwardly upon deployment of filter 10 when it is in a radially compressed form. Furthermore, the free ends of each leg 20 facilitate the later retrieval, if desired, of filter 10 from a blood vessel or other body passage since growth of cells over leg 20 will not mechanically interfere with the withdrawal of leg 20 in a generally axial direction.

Second filter basket 16 has a plurality of legs 30 which are interconnected by a plurality of struts 32 extending therebetween. Struts 32 provide increased filtering capability to basket 16 and also support radially inward segments of legs 30. Each leg 30 has a generally axially extending segment 34 and a free end 36 to facilitate the removal of the leg from any tissue which has grown over the leg after filter 10 has been emplaced in an associated blood vessel or other body passage. Axially extending segments of legs 30 extend generally parallel to each other and to the longitudinal axis of filter 10.

The unique combination of different basket structures in medical filter 10 combines the advantages of both filter basket designs while avoiding difficulties with entanglement during deployment. Thus, when medical filter 10 is in compressed form, the "straight" legs 20 of filter basket 14 extend axially in parallel fashion to the spine 12 of filter 10 so that upon deployment and radially outward expansion of legs 20, there is little chance of any entanglement of legs 20 with spine 12 or with each other. Filter basket 16 has a filter structure which includes struts 32 and, hence, increased filtering capability, e.g., clot capturing efficiency. When radially expanded from compressed form, struts 32 are free of spine 12 so that there can be no entanglement with spine 12. Thus, filter 10 enjoys advantages of improved filtering capability without increased risk of entanglement of filter elements. Furthermore, filter 10 has improved resistance to occlusion since basket 14 does not employ struts between legs 20.

In a preferred use of medical filter 10, it is contemplated that filter 10 will be deployed in the vena cava with retrieval element 18 at the caudal end of the filter. In this orientation, filter basket 14 can serve as a relatively large pore filter and filter basket 16 can serve as a relatively small pore filter. Such an arrangement will improve the filters resistance to occlusion. Of course, the design has no closed elements which will be in contact with a vessel wall and is thus designed for long term retrievability.

Retrieval element 18 can be a single hook as shown in the Figure 1 or can have a T-shape with twin hooks or any other suitable shape which can operatively interact with a snare device. It will be appreciated by those skilled in the art that hooks and snares are well-known and that the particular retrieval means employed is subject to variation within the scope of the present invention.

While filter 10 is suitable for permanent placement in a vessel or body passage, filter 10 may be readily removed or retrieved if retrieval is desired. Retrieval of filter 10 may be accomplished by means of a conventional retrieval catheter which may be inserted from the appropriate direction toward the end of filter 10 having retrieval element 18. After snaring retrieval element 18 with the snare, filter 10 is drawn into the lumen of the retrieval catheter and retrieved in a conventional manner. It will be appreciated that the free ends of legs 20 and 30 their direction of extension allow their removal with minimal trauma even if they have become incorporated into tissue of an associated vessel.

Filter 10 may be made of any suitable material using a variety of methods. Nitinol is a preferred material but elgiloy, cobalt chromium, stainless steel or suitable plastic are examples of other materials that may be used so long as the material has the desired characteristics of strength, resilience, flexibility, biocompatibility and endurance and is suitable for the particular manufacturing technique employed. It is, of course, required that the material employed be capable of expanding to the desired shape upon ejection from the delivery catheter. Thus, the material must also be sufficiently resilient to accomplish both compression in the delivery catheter and expansion upon ejection from the catheter.

Suitable methods of manufacture of medical filter 10 include cutting a pattern into a tube to enable expansion of the tube into the desired body and struts. Another suitable method is forming the struts and body from separate strips or wires and then joining the respective parts together by suitable methods which are well known in the art.

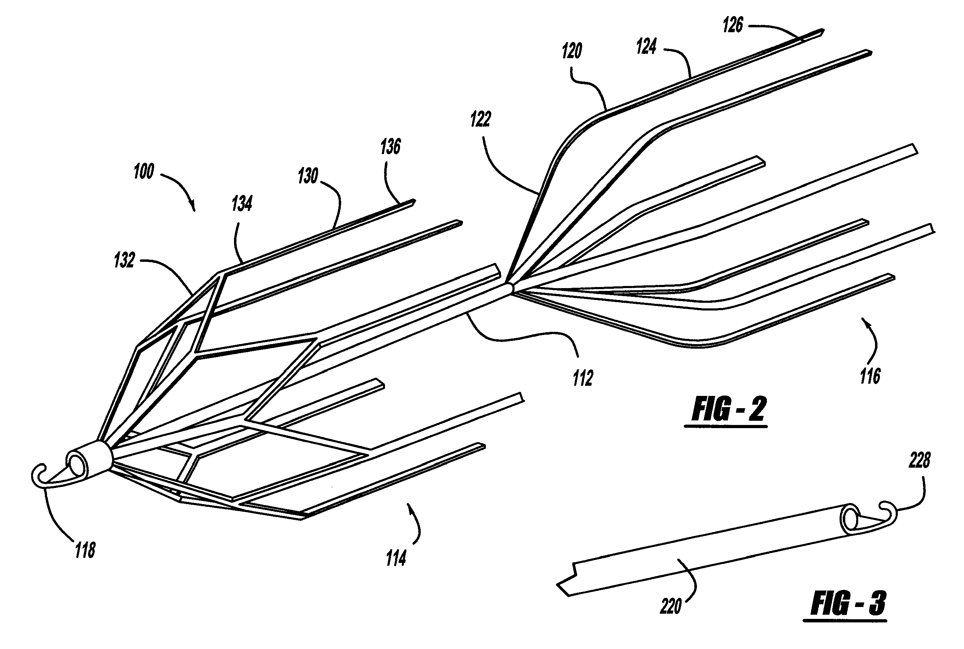

Now referring to Figure 2, an alternative preferred embodiment of the present invention is shown and indicated generally by the numeral 100. Medical filter 100 has a spine 112 with first filter basket 114 and second filter basket 116 and with retrieval element 118. First filter basket 114 has legs 130 interconnected by struts 132. Legs 130 have axially extending segments 134 and free ends 136. Second filter basket 116 has legs 120 with generally radially outwardly and axially extending segments 122, generally axially extending segments 124 and free ends 126. First filter basket 114 is of a structure analogous to first filter basket 14 of medical filter 10 and second filter basket 116 is of a structure analogous to second filter basket 16 of medical filter 10. But in medical filter 100 the arrangement of the two filter baskets has been reversed along spine 112 so that first filter basket 114 is proximal to retrieval element 118 and second filter basket 116 is distal to retrieval element 112.

A further variation of the present invention is shown in Figure 3 which illustrates a variation in the leg structure of the filter of Figure 2. Thus, leg 220 is shown with reentrantly bent end portion 228 so that upon deployment in a blood vessel or other body passage, the wall of the vessel or passage will be engaged by the reentrently bent end portion to prevent migration. This design allows the filter to be implanted with the retrieval element at the cranial end of the filter and allows for retrieval from a jugular approach. Retrieval from a jugular approach is preferred by some physicians.

While preferred embodiments of the present invention have been specifically described above, it will be appreciated by those skilled in the art that the present invention is subject to variations and modifications. For example, the filter may be cut from a single tube and have a head which is integral, i.e., one piece, with the struts. The filter may be tube-based or wire based or a combination. The second filter basket may be modified by modifying the number of struts and/or the curvature of the struts, which may be straight, curved, helical or a complex curve in any plane. More than two filter baskets may be employed in the filter and the spine may carry more than one retrieval element and the type of retrieval element is subject to variation and modification without departing from the scope of this invention. For example, the retrieval hook may be modified by changing the angle of the hook and/or by modifying the number of tines for engaging the retrieval snare. These and other modifications are contemplated to be within the scope of the present invention which is intended to be limited only by the following claims.

## Claims

1. A radially expandable and compressible, elongated medical filter having a longitudinal axis, said filter intended for placement inside a tubular body passage of a patient and comprising in expanded form:
A. a spine extending along the longitudinal axis of the filter and having a retrieval hook at one end thereof;
B. a first filter basket attached to said spine proximal to said retrieval hook and comprising a plurality of discrete legs, each leg having one end secured to said spine and a free end; and
C. a second filter basket attached to said spine distal to said retrieval hook and longitudinally spaced from said first filter basket and comprising a plurality of legs, each of said legs having one end secured to said spine and a free end, and further comprising a plurality of interconnecting struts extending between the legs proximate to said ends connected to said spine.

2. A radially expandable and compressible, elongated medical filter having a longitudinal axis, said filter intended for placement inside a tubular body passage of a patient and comprising in expanded form:
A. a spine extending along the longitudinal axis of the filter and having a retrieval hook at one end thereof;
B. a first filter basket attached to said spine distal to said retrieval hook and comprising a plurality of discrete legs, each leg having one end secured to said spine and a free end; and
C. a second filter basket attached to said spine proximal to said retrieval hook and longitudinally spaced from said first filter basket and comprising a plurality of legs, each of said legs having one end secured to said spine and a free end, and further comprising a plurality of interconnecting struts extending between the legs proximate to said ends connected to said spine.

3. A radially expandable and compressible, elongated medical filter having a longitudinal axis, said filter intended for placement inside a tubular body passage of a patient and comprising in expanded form:
A. a spine extending along the longitudinal axis of the filter and having a retrieval element at one end thereof;
B. a first filter basket attached to said spine and comprising a plurality of discrete legs with free ends; and
C. a second filter basket attached to said spine longitudinally spaced from said first filter basket and comprising a plurality of legs with free ends and further with interconnecting struts extending between the legs.

4. The medical filter of any one of claims 1 to 3, wherein said filter is comprised ofNitinol.

5. The medical filter of any one of claims 1 to 3, wherein said filter is comprised of stainless steel.

6. The medical filter of any one of claims 1 to 3, wherein said legs are comprised of wire.

7. The medical filter of any one of claims 1 to 3, wherein said legs are cut from tubing.

8. The medical filter of claim 3 wherein at least some of the legs of one of the first and second filter baskets have reentrantly bent end segments.

9. The medical filter of claim 3 wherein the first filter basket is attached to the spine proximate to the retrieval element while the second filter basket is attached to the spine distal to the retrieval element.

10. The medical filter of claim 3 wherein said retrieval element is a hook.

11. The medical filter of claim 3 wherein said second filter basket is secured to a longitudinal end of said spine.

12. The medical filter of claim 3 wherein said second filter basket is secured to said spine at a point spaced longitudinally from said first filter basket.

13. The medical filter of claim 3 wherein each of said legs has a segment extending generally parallel to the longitudinal axis of said filter.

14. The medical filter of claim 3 wherein first filter basket is attached to the spine distal to the retrieval element while the second filter basket is attached to the spine proximal to the retrieval element.
